# EUROPEAN PATENT APPLICATION

(11) **EP 3 693 462 A1**
(43) Date of publication of application: **12.08.2020**
(21) Application number: 20151461.9
(22) Date of filing: 17.07.2012
(51) Int. Cl.: C12N 15/113, A61K 31/7105, A61K 31/711, A61P 1/04, A61P 29/00, A61P 37/00

(54) **MUCOSAL HEALING PROMOTER**

(62) Divisional of application: 16184500.3
(71) Applicant: Stelic Institute&Co., Tokyo 106-0044 (JP)
(72) Inventor: YONEYAMA, Hiroyuki, Minato-ku, Tokyo 106-0044 (JP); FUJII, Masato, Minato-ku, Tokyo 106-0044 (JP)
(74) Representative: Grünecker Patent- und Rechtsanwälte PartG mbB

(57) **Abstract**

From experiments using colitis model mice, the present inventors discovered that siRNAs that suppress the CHST15 gene expression have a therapeutic effect against Crohn's disease or ulcerative colitis. Specifically, the present inventors discovered that the siRNAs which suppress the CHST15 gene expression can serve as an agent for promoting mucosal healing, in particular, an agent for treating Crohn's disease or ulcerative colitis, and thereby completed the present invention.

SEQUENCE LISTING

<110>  STELIC INSTITUTE & CO.

<120>  Mucosal healing promoter

<130>  EP98263II-FZ200

<140>  not yet assigned
<141>  2012-07-17

<150>  EP16184500.3
<151>  2012-07-17

<150>  EP12881408.4
<151>  2012-07-17

<150>  PCT/JP2012/068068
<151>  2012-07-17

<160>  6

<170>  PatentIn version 3.5

<210>  1
<211>  25
<212>  RNA
<213>  Artificial Sequence

<220>
<223>  An artificially synthesized siRNA sequence

<400>  1
ggagcagagc aagaugaaua caauc                                          25


<210>  2
<211>  25
<212>  RNA
<213>  Artificial Sequence

<220>
<223>  An artificially synthesized siRNA sequence

<400>  2
gauuguauuc aucuugcucu gcucc                                          25


<210>  3
<211>  27
<212>  RNA
<213>  Artificial Sequence

<220>
<223>  An artificially synthesized siRNA sequence

<400>  3
ggagcagagc aagaugaaua caaucag                                        27


<210>  4
<211>  27
<212>  RNA

<213>  Artificial Sequence

<220>
<223>  An artificially synthesized siRNA sequence

<400>  4
gauuguauuc aucuugcucu gcuccau                                          27


<210>  5
<211>  4713
<212>  DNA
<213>  Homo sapiens


<220>
<221>  CDS
<222>  (514)..(2199)

<400>  5
   ggaaatctgg cattttttaa agtttgcgcc ccacaaagag gaaatattcc aaaggtactc      60
   aggatgtaaa aggggagatc ttcacagatg cctccgtgga tggcatggca atccatccat     120
   caatgagaag accatgattt cttttaattt tctgtgtgtt tccacattcc ccagtgagaa     180
   ttcttccacc tttttttgtg ccatgggaaa aacctgaagg gcaggcagag ctgctcccga     240
   acttgtgacc ttctctgagg ttgcagcggc tcttgtagaa catgactctg ggacatcact     300
   tccttttgtt ttctttcgga gctgaaccaa agaatgtgca ccctctttct ctagtgctgt     360
   ggtgtctgct tatttttgta tttgtgcttt ccatccatct tctgtgatca caaggcattc     420
   ttaaggtttt ctagcacgac ttgcggacat ccagactcgt gggggcccca cccatggctc     480
   ggtaagccag cagcccaggg cactggcact acc atg agg cac tgc att aat tgc      534
                                        Met Arg His Cys Ile Asn Cys
                                        1               5
   tgc ata cag ctg tta ccc gac ggc gca cac aag cag cag gtc aac tgc      582
   Cys Ile Gln Leu Leu Pro Asp Gly Ala His Lys Gln Gln Val Asn Cys
           10                  15                  20
   caa ggg ggc ccc cat cac ggt cac cag gcg tgc ccc acg tgc aaa gga      630
   Gln Gly Gly Pro His His Gly His Gln Ala Cys Pro Thr Cys Lys Gly
       25                  30                  35
   gaa aac aaa att ctg ttt cgt gtg gac agt aag cag atg aac ttg ctt      678
   Glu Asn Lys Ile Leu Phe Arg Val Asp Ser Lys Gln Met Asn Leu Leu
   40                  45                  50                  55
   gct gtt ctc gaa gtg agg act gaa ggg aac gaa aac tgg ggt ggg ttt      726
   Ala Val Leu Glu Val Arg Thr Glu Gly Asn Glu Asn Trp Gly Gly Phe
                   60                  65                  70
   ttg cgc ttc aaa aag ggg aag cga tgt agc ctc gtt ttt gga ctg ata      774
   Leu Arg Phe Lys Lys Gly Lys Arg Cys Ser Leu Val Phe Gly Leu Ile
               75                  80                  85
   ata atg acc ttg gta atg gct tct tac atc ctt tct ggg gcc cac caa      822
   Ile Met Thr Leu Val Met Ala Ser Tyr Ile Leu Ser Gly Ala His Gln
           90                  95                  100
   gag ctt ctg atc tca tca cct ttc cat tac gga ggc ttc ccc agc aac 870
   Glu Leu Leu Ile Ser Ser Pro Phe His Tyr Gly Gly Phe Pro Ser Asn
   105 110 115
   ccc agc ttg atg gac agc gaa aac cca agt gac aca aag gag cat cac 918
   Pro Ser Leu Met Asp Ser Glu Asn Pro Ser Asp Thr Lys Glu His His
   120 125 130 135
   cac caa tcc tct gta aat aat att tca tac atg aag gac tat cca agc 966
   His Gln Ser Ser Val Asn Asn Ile Ser Tyr Met Lys Asp Tyr Pro Ser
   140 145 150
   att aaa tta att atc aac agc atc aca act agg att gag ttc acg acc 1014
   Ile Lys Leu Ile Ile Asn Ser Ile Thr Thr Arg Ile Glu Phe Thr Thr
   155 160 165
   aga cag ctc cca gac tta gaa gac ctt aag aag cag gag ttg cat atg 1062
   Arg Gln Leu Pro Asp Leu Glu Asp Leu Lys Lys Gln Glu Leu His Met
   170 175 180
   ttt tca gtc atc ccc aac aaa ttc ctt cca aac agt aag agc ccc tgt 1110
   Phe Ser Val Ile Pro Asn Lys Phe Leu Pro Asn Ser Lys Ser Pro Cys
   185 190 195
   tgg tac gag gag ttc tcg ggg cag aac acc acc gac ccc tac ctc acc 1158
   Trp Tyr Glu Glu Phe Ser Gly Gln Asn Thr Thr Asp Pro Tyr Leu Thr
   200 205 210 215
   aac tcc tac gtg ctc tac tcc aag cgc ttc cgc tcc acc ttc gac gcc 1206
   Asn Ser Tyr Val Leu Tyr Ser Lys Arg Phe Arg Ser Thr Phe Asp Ala
   220 225 230
   ctg cgc aag gcc ttc tgg ggc cac ctg gcg cac gcg cac ggg aag cac 1254
   Leu Arg Lys Ala Phe Trp Gly His Leu Ala His Ala His Gly Lys His
   235 240 245
   ttc cgc ctg cgc tgc ctg ccg cac ttc tac atc ata ggg cag ccc aag 1302
   Phe Arg Leu Arg Cys Leu Pro His Phe Tyr Ile Ile Gly Gln Pro Lys
   250 255 260
   tgc ggg acc aca gac ctc tat gac cgc ctg cgg ctg cac cct gag gtc 1350
   Cys Gly Thr Thr Asp Leu Tyr Asp Arg Leu Arg Leu His Pro Glu Val
   265 270 275
   aag ttc tcc gcc atc aag gag cca cac tgg tgg acc cgg aag cgc ttt 1398
   Lys Phe Ser Ala Ile Lys Glu Pro His Trp Trp Thr Arg Lys Arg Phe
   280 285 290 295
   gga atc gtc cgc cta aga gat ggg ctg cga gac cgc tat ccc gtg gaa 1446
   Gly Ile Val Arg Leu Arg Asp Gly Leu Arg Asp Arg Tyr Pro Val Glu
   300 305 310
   gat tat ctg gac ctc ttt gac ctg gcc gca cac cag atc cat caa gga 1494
   Asp Tyr Leu Asp Leu Phe Asp Leu Ala Ala His Gln Ile His Gln Gly
   315 320 325
   ctg cag gcc agc tct gca aag gag cag agc aag atg aat aca atc att 1542
   Leu Gln Ala Ser Ser Ala Lys Glu Gln Ser Lys Met Asn Thr Ile Ile
   330 335 340
   atc ggg gag gcc agt gcc tcc acg atg tgg gat aat aat gcc tgg acg 1590
   Ile Gly Glu Ala Ser Ala Ser Thr Met Trp Asp Asn Asn Ala Trp Thr
   345                 350                 355
   ttc ttc tac gac aac agc acg gat ggc gag cca ccg ttt ctg acg cag      1638
   Phe Phe Tyr Asp Asn Ser Thr Asp Gly Glu Pro Pro Phe Leu Thr Gln
   360                 365                 370                 375
   gac ttc atc cac gcc ttt cag cca aat gcc aga ctg att gtc atg ctc      1686
   Asp Phe Ile His Ala Phe Gln Pro Asn Ala Arg Leu Ile Val Met Leu
                   380                 385                 390
   agg gac cct gtg gag agg ttg tac tca gac tat ctc tac ttt gca agt      1734
   Arg Asp Pro Val Glu Arg Leu Tyr Ser Asp Tyr Leu Tyr Phe Ala Ser
               395                 400                 405
   tcg aat aaa tcc gcg gac gac ttc cat gag aaa gtg aca gaa gca ctg      1782
   Ser Asn Lys Ser Ala Asp Asp Phe His Glu Lys Val Thr Glu Ala Leu
           410                 415                 420
   cag ctg ttt gaa aat tgc atg ctt gat tat tca ctg cgc gcc tgc gtc      1830
   Gln Leu Phe Glu Asn Cys Met Leu Asp Tyr Ser Leu Arg Ala Cys Val
       425                 430                 435
   tac aac aac acc ctc aac aac gcc atg cct gtg agg ctc cag gtt ggg      1878
   Tyr Asn Asn Thr Leu Asn Asn Ala Met Pro Val Arg Leu Gln Val Gly
   440                 445                 450                 455
   ctc tat gct gtg tac ctt ctg gac tgg ctc agc gtt ttt gac aag caa      1926
   Leu Tyr Ala Val Tyr Leu Leu Asp Trp Leu Ser Val Phe Asp Lys Gln
                   460                 465                 470
   cag ttt ctc att ctt cgc ctg gaa gat cat gca tcc aac gtc aag tac      1974
   Gln Phe Leu Ile Leu Arg Leu Glu Asp His Ala Ser Asn Val Lys Tyr
               475                 480                 485
   acc atg cac aag gtc ttc cag ttt ctg aac cta ggg ccc tta agt gag      2022
   Thr Met His Lys Val Phe Gln Phe Leu Asn Leu Gly Pro Leu Ser Glu
           490                 495                 500
   aag cag gag gct ttg atg acc aag agc ccc gca tcc aat gca cgg cgt      2070
   Lys Gln Glu Ala Leu Met Thr Lys Ser Pro Ala Ser Asn Ala Arg Arg
       505                 510                 515
   ccc gag gac cgg aac ctg ggg ccc atg tgg ccc atc aca cag aag att      2118
   Pro Glu Asp Arg Asn Leu Gly Pro Met Trp Pro Ile Thr Gln Lys Ile
   520                 525                 530                 535
   ctg cgg gat ttc tac agg ccc ttc aac gct agg ctg gcg cag gtc ctc      2166
   Leu Arg Asp Phe Tyr Arg Pro Phe Asn Ala Arg Leu Ala Gln Val Leu
                   540                 545                 550
   gcg gat gag gcg ttt gcg tgg aag acg acg tga gagctgaatt gttgctgcac      2219
   Ala Asp Glu Ala Phe Ala Trp Lys Thr Thr
               555                 560
   gtgctgggcc cgccaatgcc gtcatcatca ggattttaca aatctctttg cggggaactg      2279
   tttcactcat ggtatggaaa accccaggac tctgccactc taggcacaca tgaattataa      2339
   ccattttgga atttccttcg tgatgttcga gagctcagca atggacccct cacagagctc      2399
   ctctatccga ggccattgga gaccccagtt tctcaagaat tcagctctgc tctgagcgtc      2459
   ctggagcttg gggatgcagc cagctggcct gcactgggtg tggagagaac acctagggaa      2519
   ggcagcctgg ccctgcccgc ctccgccttc tggagagcct ctgggttctg agtcagcaag 2579
   ccagaggtca tgccacaggc ctggctggaa cttacacttc acgttccctt tttttccccc 2639
   tagagatggg gtctcgccgt gttgcacaga ctgtctgtat tcaatggcta tcttcacagg 2699
   tgtgatcata ccacattcac ttctgaaaca ctcttgttgc gatcgctaac ctcactggga 2759
   cagagaaccg cagtctttcg agaatggagg ctcttcattt tttttttctc ctttactcca 2819
   aactcagccc tccagtttct tcagatgtaa accctgttaa cgtcactgtt tccaaaagga 2879
   aaaaaataag tcagtttttg gcagcacctt catctttctg acctcctcct attctgtcct 2939
   tgtggactta tgtttaacat agaaaatgaa tgcgtttaaa acaaaaccac tttctgcatt 2999
   taaccagtcc tggctctctc tctgctgcct cttcatacgt tttctcaaga acttcagttt 3059
   ataattggaa gagaaatttt tgctgttaat gccagaatga gcaacctcaa ggaattgaac 3119
   acttcttgga aaatctaggt aattcaagcc ctcatcaggt ttacaagatc atcagagaaa 3179
   cagaggattt taatttttag ttctggccgg ctacaggctc catttctctg ccttcccatt 3239
   ggaaatagtt tatttccaca ttctccactg cgtgtggtca aagttcctca cccagcaagg 3299
   gactatagat actcgtgtcc caattccaaa acacaatgca caagctgaac ttgggctgaa 3359
   cgtggcgtgt tgagatttgg aatgaggttt ctaagagccg tgttcttcat ggaattttcc 3419
   aggccacttg gcagcttggt ttaccgatgg atgggctaga gatcttgtcg tttcttggaa 3479
   gtcacaggga agattgaaga gaacgcttga gcatccttgg caacagccca ggtgggacct 3539
   ggatgaagct ttgcactcaa gtattgtcaa gggaagcttc ctgtgaacca aagttctcag 3599
   gccaaggtct cgcccaccaa agccagaaag tgcaagcacc cgtctaccca gctctaactt 3659
   gtatgtgtga gacagaccag gcttcggggg taggaggatc tgcagttgtt cagccgtctt 3719
   tctgctggtg ttgtctttct gccatcagag aagggacaca cagcccgttc gaaggtgtgc 3779
   agagggctct gagcgccagg atggccaggg ctgtttttgc tactgaagga gcgtgtgtcc 3839
   tgaactccca cttgcaggga cagtccccac cttctctata gccggcactg ggagcagccg 3899
   ccagcaggga aatctggcct gagcacaagg atgctttagg gagagatcac ttcagtgtgt 3959
   gtgtatattt atttgcagta cagtgcgcgc gtgtgtgtgt gtgtacgcgc acgtgtgggt 4019
   gagtgcgtct tctgagtggg ttctgttcag ttgctaatga ggctcctccg ctctggacac 4079
   aaccctttta tagattaatt tctctgccaa ttaacttgtc attttcagta catattttac 4139
   tattccacac caaccataat tacaacaagg gatttttctt atgcactcct atgcatgtga 4199
   ataacatgtg gtgtaattct gcttcttaca gaagtattac tgaaggtatt atttccaata 4259
   ttatttggtt tattatgcgg atctttttta tatatgcagt cccatccctt ctgtgccact 4319
   caatgccatc cagacatggt ttttccctcc aggggccttt ctctccagag ggcacttcgg 4379
   ctgcctctgc ttcctctcat tcgaggcccg gctcttgctg acagaatagg ttccgttctg 4439
   ggcggtggtt ctcgagcctg ccattcaaaa ccaaagcaaa ttggagcatt tctcacaaca 4499
   tggtattgaa gttccttttt gttctcaaaa gttgtgaccg tgttaaattg tactccctta 4559
   gtcctgtaag gtatgttaag tgaatcgcag ttacgctgta cttttattaa tatttaacat 4619
   aattaaagat ggacccataa gagtgacgcc tgtggagcgc gtgctcttcc tctgcagcca 4679
   aaaaaaaaaa aaaaaaaaaa aaaaaaaaaa aaaa 4713

<210> 6
<211> 561
<212> PRT
<213> Homo sapiens

<400> 6

Met Arg His Cys Ile Asn Cys Cys Ile Gln Leu Leu Pro Asp Gly Ala
1               5                   10                  15

His Lys Gln Gln Val Asn Cys Gln Gly Gly Pro His His Gly His Gln
            20                  25                  30

Ala Cys Pro Thr Cys Lys Gly Glu Asn Lys Ile Leu Phe Arg Val Asp
        35                  40                  45

Ser Lys Gln Met Asn Leu Leu Ala Val Leu Glu Val Arg Thr Glu Gly
    50                  55                  60

Asn Glu Asn Trp Gly Gly Phe Leu Arg Phe Lys Lys Gly Lys Arg Cys
65                  70                  75                  80

Ser Leu Val Phe Gly Leu Ile Ile Met Thr Leu Val Met Ala Ser Tyr
                85                  90                  95

Ile Leu Ser Gly Ala His Gln Glu Leu Leu Ile Ser Ser Pro Phe His
            100                 105                 110

Tyr Gly Gly Phe Pro Ser Asn Pro Ser Leu Met Asp Ser Glu Asn Pro
        115                 120                 125

Ser Asp Thr Lys Glu His His His Gln Ser Ser Val Asn Asn Ile Ser
    130                 135                 140

Tyr Met Lys Asp Tyr Pro Ser Ile Lys Leu Ile Ile Asn Ser Ile Thr
145                 150                 155                 160

Thr Arg Ile Glu Phe Thr Thr Arg Gln Leu Pro Asp Leu Glu Asp Leu
                165                 170                 175

Lys Lys Gln Glu Leu His Met Phe Ser Val Ile Pro Asn Lys Phe Leu
            180                 185                 190

Pro Asn Ser Lys Ser Pro Cys Trp Tyr Glu Glu Phe Ser Gly Gln Asn
        195                 200                 205

Thr Thr Asp Pro Tyr Leu Thr Asn Ser Tyr Val Leu Tyr Ser Lys Arg
    210                 215                 220

Phe Arg Ser Thr Phe Asp Ala Leu Arg Lys Ala Phe Trp Gly His Leu
225                 230                 235                 240

Ala His Ala His Gly Lys His Phe Arg Leu Arg Cys Leu Pro His Phe
                245                 250                 255

Tyr Ile Ile Gly Gln Pro Lys Cys Gly Thr Thr Asp Leu Tyr Asp Arg
            260                 265                 270

Leu Arg Leu His Pro Glu Val Lys Phe Ser Ala Ile Lys Glu Pro His
        275                 280                 285

Trp Trp Thr Arg Lys Arg Phe Gly Ile Val Arg Leu Arg Asp Gly Leu
    290                 295                 300

Arg Asp Arg Tyr Pro Val Glu Asp Tyr Leu Asp Leu Phe Asp Leu Ala
305                 310                 315                 320

Ala His Gln Ile His Gln Gly Leu Gln Ala Ser Ser Ala Lys Glu Gln
                325                 330                 335

Ser Lys Met Asn Thr Ile Ile Ile Gly Glu Ala Ser Ala Ser Thr Met
            340                 345                 350

Trp Asp Asn Asn Ala Trp Thr Phe Phe Tyr Asp Asn Ser Thr Asp Gly
        355                 360                 365

Glu Pro Pro Phe Leu Thr Gln Asp Phe Ile His Ala Phe Gln Pro Asn
    370                 375                 380

Ala Arg Leu Ile Val Met Leu Arg Asp Pro Val Glu Arg Leu Tyr Ser
385                 390                 395                 400

Asp Tyr Leu Tyr Phe Ala Ser Ser Asn Lys Ser Ala Asp Asp Phe His
                405                 410                 415

Glu Lys Val Thr Glu Ala Leu Gln Leu Phe Glu Asn Cys Met Leu Asp
            420                 425                 430

Tyr Ser Leu Arg Ala Cys Val Tyr Asn Asn Thr Leu Asn Asn Ala Met
        435                 440                 445

Pro Val Arg Leu Gln Val Gly Leu Tyr Ala Val Tyr Leu Leu Asp Trp
    450                 455                 460

Leu Ser Val Phe Asp Lys Gln Gln Phe Leu Ile Leu Arg Leu Glu Asp
465                 470                 475                 480

His Ala Ser Asn Val Lys Tyr Thr Met His Lys Val Phe Gln Phe Leu
                485                 490                 495

Asn Leu Gly Pro Leu Ser Glu Lys Gln Glu Ala Leu Met Thr Lys Ser
            500                 505                 510

Pro Ala Ser Asn Ala Arg Arg Pro Glu Asp Arg Asn Leu Gly Pro Met
        515                 520                 525

Trp Pro Ile Thr Gln Lys Ile Leu Arg Asp Phe Tyr Arg Pro Phe Asn
    530                 535                 540

Ala Arg Leu Ala Gln Val Leu Ala Asp Glu Ala Phe Ala Trp Lys Thr
545                 550                 555                 560

Thr

## Description

### Technical Field

The present invention relates to agents for promoting mucosal healing. Specifically, the present invention relates to agents for treating chronic inflammatory diseases such as Crohn's disease, ulcerative colitis, and Behcet's disease

### Background Art

### [New development in Crohn's disease therapy after Remicade: it has now become possible to control systemic symptoms, and the next challenge in development is to control local lesions (digestive damage) = intestinal/mucosal healing]

Crohn's disease (CD) is a non-specific inflammatory disease in which discontinuous inflammation and ulceration occurs throughout the gastrointestinal tract. CD is a rare disease affecting 400,000 to 600,000 people in North America, 600,000 people in Europe, and about 30,000 people in Japan. The number of CD patients is growing due to lifestyle changes (Non-patent Document 1). CD causes inflammation and ulceration throughout the gastrointestinal tract, and the QOL is considerably impaired due to various clinical manifestations such as diarrhea, abdominal pain, fever, anemia associated with melena, weight loss, decrease in physical strength, and malaise. The inflammatory lesion in CD begins within the mucosa, and spreads to the submucosa and then to the muscularis propria; and its characteristics are transmural granulomatous inflammation, edema, and intestinal wall thickening due to fibrosis (Non-patent Documents 2 to 4).

Treatment of CD begins with agents based on anti-inflammatory and immunosuppressive mechanisms, *i.e.,* 5-ASA formulations, oral steroids, and immunosuppressive agents; and in recent years anti-TNF formulations have also been used (Non-patent Document 5). By the decrease in the Crohn's Disease Activity Index (CDAI) which is used to evaluate the CD clinical activity, it has become possible to control CD symptoms. However, there are many cases of residual local active lesions revealed by endoscopic examination even in patients who have been diagnosed with mild CD or are considered to be in remission based on CDAI; and such lesions may become an origin of stricture induced by recurrent inflammation and fibrosis (Non-patent Documents 2, 3, 6, and 7). It is an established concept that CD is progressive digestive damage (Non-patent Documents 4, 6, and 7). Also in terms of the therapeutic strategy, the focus has shifted from controlling symptoms to healing endoscopic lesions to (1) achieve long-term intestinal protection and (2) avoid surgical operations for intestinal strictures (Non-patent Documents 6 to 11). In particular, it has been reported that intestinal healing (IH) or mucosal healing (MH) at the endoscopic level is a factor that influences prognosis in CD patients (Non-patent Documents 6 to 11); and improvement in the simple endoscopic score for CD (SES-CD) which is used to assess the endoscopic CD activity has become a novel criterion for assessing the effectiveness of CD therapy. The MH effect of existing agents in CD patients is reported to be almost none by 5-ASA formulations and oral steroidal agents, 16.5% by immunosuppressants and 30.1% by anti-TNF formulations (Non-patent Documents 7 and 11). Remicade contributes to the addition of medical knowledge that it is important to find a way to heal endoscopic lesions (IH/MH) which cannot be sufficiently controlled by systemic agents; and this can be said to identify urgent challenges for novel agents against CD.

### [Novel therapeutic strategy aimed at IH/MH: from the viewpoint of clinical pathology - Therapy is started by identifying sites of chronicity and fibrotic lesions at an early stage]

The pathological cause of persistent endoscopic lesions is still not fully understood. However, the difficulty of healing endoscopic lesions in CD is ascribed to the fact that fibrosis and ulcer exist at the same time. During the process of repairing damaged tissues, one of the factors is persistence of inflammatory lesions with ulceration and fibrosis caused by inadequate repair through fibrosis (fibrotic healing) which is induced instead of adequate MH (Non-patent Documents 2, 3, and 12). It is predicted that existing systemic agents that are based on inflammatory/immune mechanisms might not produce a sufficient effect against local lesions described above when used alone. Pathologically, such lesions might persist by repeating damage and repair (Non-patent Document 3), and in CD they are diagnosed by endoscopic examination as aphtha (erosion with erythema and small edematous bumps) and ulcers, lesions with edematous narrowing of the lumen. Furthermore, technologies that enable detailed real-time observation, such as magnification endoscopy and confocal endomicroscopy, have become increasingly popular in recent years; and thus the environment is ready for making early treatment possible.

It was demonstrated that the excessive activation of fibroblasts accumulated at lesion sites from an early period of inflammation shifts the balance to fibrotic healing. Thus, "suppression of fibroblast activation" has been drawing attention as a novel target in therapy against inflammation-driven intestinal fibrosis in inflammatory bowel diseases (Non-patent Documents 2 and 3). The endoscopic lesions that could not be sufficiently treated with merely the existing agents based on inflammatory/immune mechanisms are a pathological condition where ulceration and fibrosis occur concomitantly. This is the reason why therapy targeting fibrosis/fibroblasts has surfaced as a novel therapeutic strategy for CD.

### [Novel therapeutic strategy aimed at IH/MH: from the scientific viewpoint - CHST-CS pathway]

Carbohydrate sulfotransferase 15 (CHST15), i.e., a glycosaminoglycan sulfotransferase gene, is a type II Golgi transmembrane protein that synthesizes highly sulfated chondroitin sulfate-E (CS-E) by transferring sulfate to position 6 of the GalNAc(4SO₄) residue in chondroitin sulfate-A (CS-A) (Non-patent Documents 13 and 14). It has been reported that in CD patients, synthesis of highly sulfated chondroitin sulfate (CS) is significantly increased in active lesions of the large intestine (Non-patent Document 15); highly sulfated CS-E binds to type V collagen which is increased in the thickened submucosa of CD patients (Non-patent Documents 16 and 17); and highly sulfated CS-E enhances the collagen fiber (fibril) formation (Non-patent Document 18). This suggests that CS-E is involved in the maintenance and expansion of local fibrotic lesions. Furthermore, highly sulfated CS-E has been reported to bind to CD44 which is a molecule involved in fibroblast adhesion; chemokines MCP-1 and SDF-1 which are involved in fibroblast migration; and PDGF and TGF-β which are involved in fibroblast proliferation (Non-patent Document 19). The above finding suggests that highly sulfated CS-E is also involved in the colonization and activation of fibroblasts by locally enriching these molecules at submucosal sites.

The present inventors revealed that the CHST15 protein is produced excessively at fibrotic sites in CD patients and that CHST15 is involved in intestinal fibrosis in a colitis animal model with intestinal fibrosis (Non-patent Document 20 and 21). Fibrosis was reduced when overly sulfated CS was removed by chondroitinase ABC or a chondroitin desulfating enzyme (Patent Document 1, and Non-patent Documents 20 and 21). From this finding, the present inventors conceived that regulation of the causative CHST15 production is a promising target against fibrosis in the gastrointestinal tract. There were no methods to selectively inhibit highly sulfated CS or CHST15 with conventional chemical techniques alone, and thus the present inventors proceeded with a strategy of selective inhibition by siRNA via locally injected routes.

### [Prior Art Documents]

### [Patent Documents]

[Patent Document 1] Patent No. 4585611

### [Non-patent Documents]

[Non-patent Document 1] Van Assche G, Geboes K, Rutgeerts P et al. Medical therapy for Crohn's disease strictures. Inflamm Bowel Dis 10: 55-60, 2004.
[Non-patent Document 2] Rieder F. and Fiocchi C. Intestinal fibrosis in IBD-a dynamic, multifactorial process. Nature Rev Gastroenterol Hepatol 6: 228-35, 2009.
[Non-patent Document 3] Rieder F and FIocchi C. First International summit on fibrosis in intestinal inflammation: mechanisms and biological therapies. Fibrogenesis & Tissue Repair 3: 22, 2010.
[Non-patent Document 4] Peyrin-Biroulet L, Loftus EV, Colombel J, et al. Early Crohn's disease: a proposed definition for use in disease-modification trials. Gut 59: 141-147, 2010.
[Non-patent Document 5] Kozuch PL and Hanauer SB. Treatment of inflammatory bowel disease: a review of medical therapy. World J Gastroenterol 21: 354-77, 2008.
[Non-patent Document 6] Lacucci M and Ghosh S. Looking beyond symptom relief: evolution of mucosal healing in inflammatory bowel disease. Ther Adv Gastroenterol 4: 129-143, 2011.
[Non-patent Document 7] de Chambrun GP, Peyrin-Biroulet L, Lemann M et al. Clinical implications of mucosal healing for the management of IBD. Nat Rev Gastroenterol Hepatol 7: 15-29, 2010.
[Non-patent Document 8] Pariente B, Cosnes J, Danese S et al. Development of the Crohn's disease digestive damage score, the Lemann score. Inflamm Bowel Dis 17: 1415-1422, 2011.
[Non-patent Document 9] Caviglia R, Ribolsi M, Rizzi M et al. Maintenance of remission with infliximab in inflammatory bowel disease: Efficacy and safety long-term follow up. World J Gastroenterol 13: 5238-5244, 2007.
[Non-patent Document 10] Baert F, Moortgat L, van Assche G et al. Mucosal healing predicts sustained clinical remission in patients with early-stage Crohn's disease. Gastroenterol 138: 463-468, 2010.
[Non-patent Document 11] Colombel JF, Sandborn WJ, Reinisch W et al. Infliximab, azathioprine, or combination therapy for Crohn's disease. N Eng J Med 362: 1383-1395, 2010.
[Non-patent Document 12] Wynn TA. Common and unique mechanisms regulate fibrosis in various fibroproliferative diseases. J Clin Invest 117: 524-529, 2007.
[Non-patent Document 13] sulfotransferase involved in the biosynthesis of chondroitin sulfate E in the bone marrow derived mast cells. Biochemical Biophysica Acta 1780: 687-95, 2008.
[Non-patent Document 14] Habuchi O, Moroi R, Ohtake S, et al. Enzymatic synthesis of chondroitin sulfate E by N-acetylgalactosamine 4-sulfate 6-O-sulfotransferase purified from squid cartilage. Anal Biochem 310: 129-36, 2002.
[Non-patent Document 15] Belmiro CLR, Souza HSP, Elia CCS et al. Biochemical and immunohistochemical analysis of glycosaminoglycan in inflamed and non-inflamed intestinal mucosa of patients with Crohn's disease. In J Colorectal Dis 20: 295-304, 2005.
[Non-patent Document 16] Takagaki K, Munakata H, Kakizaki I et al. Domein structure of chondroitin sulfate E octasaccharides binding to type V collagens. JBC 277: 8882-8889, 2002.
[Non-patent Document 17] Graham MF, Diegelmann RF, Elson CO et al. Collagen content and types in the intestinal strictures of Crohn's disease. Gastroenterol 94: 257-265, 1988.
[Non-patent Document 18] Kvist AJ, Hohnson AE, Morgelin M et al. Chondroitin sulfate perlecan enhances collagen fibril formation. JBC 281: 33127-33139, 2006.
[Non-patent Document 19] Yamada S and Sugahara K. Potential therapeutic Application of chondroitin sulfate/dermatan sulfate. Current Drug Discovery Technologies 5: 289-301, 2008.
[Non-patent Document 20] Kiryu H, Terai G, Imamura O et al. A detailed investigation of accessibilities around target sites of siRNA and miRNAs. Bioinformatics 2011 Apr 29. [Epub ahead of print]
[Non-patent Document 21] Suzuki K, Fujii M, Yoneyama H et al. The development of the therapeutic agent utilizing RNA interference in the colon of stenosis of Crohn's disease. J Jpn Soc Gastroenterol 107 (347): A29, 2010.

### Summary of the Invention

### [Problems to be Solved by the Invention]

An objective of the present invention is to provide agents for promoting mucosal healing, in particular, novel agents for treating Crohn's disease and ulcerative colitis.

### [Means for Solving the Problems]

RNA interference (RNAi) is one of the biological defense mechanisms that have evolved to protect cells from viral infections. In this mechanism, the unique viral RNA structure is recognized, leading to the induction of RNAi activation which enables degradation of the entire viral RNA. As a result, RNAi produces the effect of recovering infected cells from viral infections. The present inventors synthesized siRNAs having a unique structure. The siRNAs of the present invention (anti-CHST15 siRNA) are 27mer siRNAs which have been designed to specifically suppress expression of the human CHST15 gene involved in CHST15 production. The 27mer siRNA duplex very strongly suppresses the expression of the gene in the order of nM or pM. The anti-CHST15 siRNA of the present invention is a synthetic siRNA comprising a sequence of the CHST15 mRNA, *i.e.,* a sequence complementary to the sequence region of human CHST15. The antisense strand serves as a guide sequence for RNAi function. The siRNA of the present invention specifically recognizes and degrades human CHST15, and blocks expression of the gene involved in the production of glycosaminoglycan sulfotransferase (CHST15 protein). As a result, there is no transfer of sulfate groups to CS, and without activation of fibroblasts, fibrosis is assumed to be suppressed.

Through experiments using colitis model mice, the present inventors revealed that siRNAs which suppress the CHST15 gene expression produce a therapeutic effect against Crohn's disease or ulcerative colitis. Namely, the present inventors discovered that siRNAs that suppress the CHST15 gene expression could be used as an agent for treating Crohn's disease or ulcerative colitis, and thereby completed the present invention.

More specifically, the present invention provides [1] to [4] below:
[ 1] an siRNA that suppresses expression of the CHST15 gene, which comprises a structure in which an RNA comprising the nucleotide sequence of SEQ ID NO: 1 is hybridized to an RNA comprising a complementary sequence thereof.
[ 2] the siRNA of [1], which has a structure with an overhang of one or more nucleic acids at one end;
[ 3] A DNA vector capable of expressing the siRNA of [1] or [2];
[ 4] an agent for promoting mucosal healing, which comprises as an active ingredient the siRNA of [1] or [2] or the DNA of [3];
[ 5] an agent for treating Crohn's disease or ulcerative colitis, which comprises as an active ingredient the siRNA of [1] or [2] or the DNA of [3]; and
[ 6] a pharmaceutical composition for wound healing, mucosal healing, or ulcer healing, which comprises as an active ingredient the siRNA of [1] or [2] or the DNA of [3].

Furthermore, the present invention relates to:
[ a] a method for promoting mucosal healing or a method for treating Crohn's disease or ulcerative colitis, which comprises the step of administering a subject with an siRNA having a structure wherein an RNA comprising the nucleotide sequence of SEQ ID NO: 1 is hybridized to an RNA comprising a complementary sequence thereof (where the subject includes mammals such as humans and nonhuman mammals, and preferably includes patients with Crohn's disease or ulcerative colitis);
[ b] the use of an siRNA having a structure wherein an RNA comprising the nucleotide sequence of SEQ ID NO: 1 is hybridized to an RNA comprising a complementary sequence thereof, in the production of an agent for promoting mucosal healing or an agent for treating Crohn's disease or ulcerative colitis;
[ c] an siRNA having a structure wherein an RNA comprising the nucleotide sequence of SEQ ID NO: 1 is hybridized to an RNA comprising a complementary sequence thereof, for promoting mucosal healing or for treating (for use in the treatment of) Crohn's disease or ulcerative colitis; and
[ d] a process for manufacturing an agent for promoting mucosal healing or an agent for treating Crohn's disease or ulcerative colitis, wherein the agent contains an siRNA that suppresses the CHST15 gene expression and has a structure wherein an RNA comprising the nucleotide sequence of SEQ ID NO: 1 is hybridized to an RNA comprising a complementary sequence thereof.

### Brief Description of the Drawings

Fig. 1 is a schematic diagram showing the experimental design of the present invention.
Fig. 2 is a graph showing the effect of an anti-CHST15 siRNA of the present invention on the expression of the CHST15 gene in the large intestine. Each point represents mean and SD (n = 2 to 6). N indicates normal mice and C indicates the negative control group.
Fig. 3 is a graph showing the effect of an anti-CHST15 siRNA of the present invention on the level of hydroxyproline in the large intestine (Day 19). N indicates normal mice and C indicates the negative control group.
Fig. 4 is a graph showing the effect of an anti-CHST15 siRNA of the present invention on the segmental SES-CD score. The difference between the anti-CHST15 siRNA administration group and the negative control group on Day 19 was p < 0.01 (***) (Bonferroni multiple comparison test).

### Mode for Carrying Out the Invention

Hereinbelow, the present invention is illustrated in detail.

The present inventors discovered that suppression of the CHST15 (carbohydrate sulfotransferase 15) gene expression produces a therapeutic effect against Crohn's disease or ulcerative colitis, or a wound healing effect, mucosal healing effect, or ulcer healing effect. More specifically, the present inventors discovered RNA molecules (siRNAs) that produce a therapeutic effect against Crohn's disease or ulcerative colitis, or a wound healing effect, mucosal healing effect, or ulcer healing effect via suppression of the CHST15 gene expression by RNAi effect.

CHST15 of the present invention is also referred to as N-acetylgalactosamine 4-sulfate 6-O sulfotransferase (GalNAc4S-6ST).

First, the present invention provides siRNAs (including shRNAs) that suppress the CHST15 gene expression by an RNAi effect. The RNAs have a therapeutic effect against Crohn's disease or ulcerative colitis, or a wound healing effect, mucosal healing effect, or ulcer healing effect.

The CHST15 gene of the present invention is not particularly limited and is usually derived from animals, preferably from mammals, and more preferably from humans.

More specifically, RNAs capable of suppressing expression of the CHST15 gene of the present invention by an RNA interference (RNAi) effect (herein, also simply referred to as "siRNA of the present invention") include RNAs comprising the nucleotide sequence of any one of SEQ ID NOs: 1 to 4. Furthermore, in a preferred embodiment, the siRNA of the present invention includes double-stranded RNAs (siRNAs) in which either strand comprises the nucleotide sequence of SEQ ID NO: 1 or 2.

The present invention provides double-stranded RNAs (siRNAs) capable of suppressing the CHST15 gene expression by an RNAi effect, which have a structure in which an RNA comprising the nucleotide sequence of SEQ ID NO: 1 is hybridized to an RNA comprising a complementary sequence thereof.

The siRNA of the present invention comprising the nucleotide sequence of SEQ ID NO: 1 (5'-GGAGCAGAGCAAGAUGAAUACAAUC-3') includes, for example, RNA molecules having a structure described below. 
5’ -GGAGCAGAGCAAGAUGAAUACAAUC-3’ (SEQ ID NO: 1)
IIIIIIIIIIIIIIIIIIIIIIIII
3’ -CCUCGUCUCGUUCUACUUAUGUUAG-5’ (SEQ ID NO: 2)
 ("I" shown above represents a hydrogen bond)

In addition, RNA molecules described above in which either end has a closed structure, for example, siRNAs having a hairpin structure (a stem-loop structure) (shRNAs), are also included in the present invention. Specifically, molecules capable of forming an intramolecular double-stranded RNA structure are also included in the present invention.

For example, molecules such as 5'-GGAGCAGAGCAAGAUGAAUACAAUC (SEQ ID NO: 1) (xxxx)n GAUUGUAUUCAUCUUGCUCUGCUCC (SEQ ID NO: 2)-3' are also included in the present invention (where "(xxxx)n" represents a polynucleotide containing nucleotide residues of arbitrary type and number).

Furthermore, molecules such as 5'-GGAGCAGAGCAAGAUGAAUACAAUCAG (SEQ ID NO: 3) (xxxx)n GAUUGUAUUCAUCUUGCUCUGCUCCAU (SEQ ID NO: 4)-3' are also included in the present invention.

The nucleotides in the siRNAs of the present invention do not necessarily need to be all ribonucleotides (RNAs). Specifically, one or more of the ribonucleotides constituting the siRNAs of the present invention may be corresponding deoxyribonucleotides, as long as the molecules have the function of suppressing expression of the CHST15 gene. The term "corresponding" means that although the sugar moieties are structurally differently, the type of the base (adenine, guanine, cytosine, or thymine (uracil)) is the same. For example, a deoxyribonucleotide corresponding to a ribonucleotide with adenine refers to a deoxyribonucleotide with adenine. The term "or more" described above is not particularly limited, but preferably refers to a small number of about two to five.

In general, RNAi refers to a phenomenon in which the destruction of a target gene mRNA is induced and the target gene expression is inhibited by introduction into cells of a double-stranded RNA that comprises a sense RNA having a sequence homologous to the mRNA sequence of the target gene and an antisense RNA having a sequence complementary to the sense RNA. Typically, a double-stranded RNA having an RNAi effect comprises a sense RNA having a sequence homologous to a contiguous RNA region in the mRNA of the target gene whose expression is to be suppressed and an antisense RNA having a sequence complementary to the sense RNA.

A preferred embodiment of the present invention is a double-stranded RNA that is capable of suppressing the CHST15 gene expression by an RNAi effect, and has a structure in which an RNA comprising the nucleotide sequence of SEQ ID NO: 1 is hybridized to an RNA comprising a sequence complementary to the RNA. Thus, the present invention provides siRNAs which suppress the CHST15 gene expression and contain a structure in which an RNA comprising the nucleotide sequence of SEQ ID NO: 1 is hybridized to an RNA comprising a complementary sequence thereof. More specifically, the siRNAs include nucleic acid molecules having a structure in which the RNA of SEQ ID NO: 1 is hybridized to the RNA of SEQ ID NO: 2.

In a preferred embodiment, the siRNA of the present invention is preferably a double-stranded RNA (siRNA) that is capable of suppressing the CHST15 gene expression by an RNAi effect, and has a structure in which an RNA comprising the nucleotide sequence of SEQ ID NO: 1 is hybridized to an RNA comprising a complementary sequence thereof. The present invention includes also double-stranded RNAs (RNA-DNA hybrid molecules) that have a structure in which, for example, one or more RNAs or DNAs are added or deleted at the ends of the double-stranded RNA.

The siRNAs of the present invention may be molecules having an overhang of several nucleotides at the ends. There is no particular limitation as to the length and sequence of nucleotides forming this overhang. The overhang may be DNA or RNA. The overhang is preferably, for example, an overhang of two nucleotides. An example is a double-stranded RNA having an AG or AU overhang at the 3 '-end. The double-stranded RNAs of the present invention also include molecules in which the nucleotides forming the overhang are DNAs.

In a preferred embodiment, the siRNA of the present invention is, for example, the siRNA molecule shown below in which the nucleotides at the 3'-end overhang part are AG and AU (which corresponds to the "anti-CHST15 siRNA" in the Examples).

5' -GGAGCAGAGCAAGAUGAAUACAAUCAG-3'    (SEQ ID NO: 3)
IIIIIIIIIIIIIIIIIIIIIIIII
3' -UACCUCGUCUCGUUCUACUUAUGUUAG-5'    (SEQ ID NO: 4)

Thus, in a preferred embodiment, the siRNA of the present invention is an siRNA molecule having a structure in which the RNA of SEQ ID NO: 3 is hybridized to the RNA of SEQ ID NO: 4.

Based on the nucleotide sequences disclosed herein, those skilled in the art can appropriately generate the siRNAs of the present invention. Specifically, the double-stranded RNAs of the present invention can be generated based on the nucleotide sequence of any one of SEQ ID NOs: 1 to 4. When one of the strands (for example, the nucleotide sequence of SEQ ID NO: 1) is determined, those skilled in the art can easily know the nucleotide sequence of the other strand (complementary strand). Those skilled in the art can appropriately generate the siRNAs of the present invention using commercially available nucleic acid synthesizers. Moreover, general custom synthesis services may be used to synthesize desired RNAs.

The siRNAs of the present invention (for example, double-stranded RNA molecules in which one of the strands has the nucleotide sequence of any one of SEQ ID NOs: 1 to 4) have an effect of promoting mucosal healing, in particular, an effect of treating Crohn's disease or ulcerative colitis. Thus, the present invention provides agents for promoting mucosal healing or agents for treating Crohn's disease or ulcerative colitis, which comprise an siRNA of the present invention as an active ingredient. Furthermore, the siRNAs of the present invention have a wound healing effect, mucosal healing effect, or ulcer healing effect. Thus, the present invention provides agents for promoting mucosal healing, agents for healing wounds, agents for healing the mucosal membrane, and agents for healing ulcers, which comprise an siRNA of the present invention as an active ingredient. The present invention also provides pharmaceutical compositions for healing wounds, healing the mucosal membrane, or healing ulcers, which comprise an siRNA of the present invention as an active ingredient.

In the present invention, the target disease being treated includes, for example, mucosal healing, wound healing, and ulcer healing upon inflammation or injury.

The agents for treating chronic inflammatory diseases of the present invention are useful as therapeutic agents for various chronic inflammatory diseases such as, for example, elastosis, scleroderma, chronic peritonitis, and retroperitoneal fibrosis in integumentary and epithelial tissues such as skin;
polymyositis, dermatomyositis, polyarteritis nodosa, soft tissue fibrosis, chronic rheumatoid arthritis, palmar fibromatosis, tendinitis, tenovaginitis, Achilles tendinitis, mycetoma pedis, and such in supportive tissues such as connective tissues and muscles;
myelofibrosis, hypersplenism, vasculitis, bradyarrhythmia, arteriosclerosis, obstructive thrombotic angiitis, nodular fibrosis, angina pectoris, dilated congestive cardiomyopathy, heart failure, restrictive cardiomyopathy, diffuse nonobstructive cardiomyopathy, obstructive cardiomyopathy, cor pulmonale, mitral stenosis, aortic valve stenosis, chronic pericarditis, endocardial fibrosis, endomyocardial fibrosis, and such in blood tissues and vascular system such as bone marrow and heart;
chronic pancreatitis, Crohn's disease, ulcerative colitis, alcoholic hepatitis, chronic hepatitis B, chronic hepatitis C, Wilson's disease, cirrhosis, viral hepatitis, Gaucher's disease, glycogen storage disease, alpha 1-antitrypsin deficiency, hemochromatosis, tyrosinemia, levulosemia, galactosemia, Zellweger syndrome, congenital hepatic fibrosis, portal hypertension, hepatic granulomatosis, Budd-Chiari syndrome, primary sclerosing cholangitis, fatty liver, nonalcoholic hepatitis, hepatic fibrosis, congenital hepatic fibrosis, alcoholic cirrhosis, viral cirrhosis, parasitic cirrhosis, toxic cirrhosis, trophopathic cirrhosis, congestive cirrhosis, hepatic sclerosis, Charcot's cirrhosis, Todd's cirrhosis, secondary biliary cirrhosis, unilobar cirrhosis, cirrhosis resulting from chronic nonsuppurative destructive cholangitis, obstructive cirrhosis, cholangiolitic cirrhosis, biliary cirrhosis, atrophic cirrhosis, postnecrotic cirrhosis, posthepatitic cirrhosis, nodular cirrhosis of the liver, mixed cirrhosis, micronodular cirrhosis, compensatory cirrhosis, decompensated cirrhosis, macronodular cirrhosis, septal cirrhosis, cryptogenic cirrhosis, periportal cirrhosis, portal cirrhosis, primary biliary cirrhosis, and such in the gastrointestinal system such as liver;
coccidioidomycosis, blastomycosis, allergic bronchopulmonary aspergillosis, Goodpasture's syndrome, pulmonary fibrosis associated with adult respiratory distress syndrome, chronic obstructive pulmonary disease, pulmonary atelectasis, pneumonia, chalicosis, asbestosis, hypersensitivity pneumonitis, idiopathic pulmonary fibrosis, lymphocytic interstitial pneumonia, Langerhans-cell granulomatosis, cystic fibrosis, pustular fibrosis, pulmonary fibrosis, idiopathic pulmonary fibrosis, fibrosing pulmonary alveolitis, interstitial fibrosis, diffuse pulmonary fibrosis, chronic interstitial pneumonia, bronchiectasis, bronchiolar fibrosis, peribronchial fibrosis, pleural fibrosis, and such in the respiratory system such as lung;
male hypogonadism, myotonic dystrophy, fibrosis such as associated with Peyronie's disease, chronic tubulointerstitial nephritis, autosomal recessive cystic kidney, myeloma kidney, hydronephrosis, rapidly progressive glomerulonephritis, nephrotoxic diseases, xanthogranulomatous pyelonephritis, sickle cell nephropathy, nephrogenic diabetes insipidus, autosomal dominant polycystic kidney disease, chronic glomerular nephritis, IgA nephropathy, renal sclerosis, focal glomerulosclerosis, membranous nephritis, membranoproliferative glomerulonephritis, chronic pyelonephritis, renal amyloidosis, polycystic kidney disease, retroperitoneal fibrosis, pathology in the kidney associated with a connective tissue disease such as lupus nephritis, diabetic nephropathy, chronic prostatitis, and urocystitis associated with schistosomiasis in the urogenital system such as kidney;
fibrotic breast disease, mammary fibroadenoma, and such; congenital torticollis, ankylosing spondylitis, spinal cord disorders such as neurofibroma and neurological dysfunction after spinal cord injury, and cranial nerve diseases such as Parkinson's disease and Alzheimer's disease in the nervous system such as spine;
retrolental fibrosis and proliferative retinopathy in the eyeball; sarcoidosis that develops systemic involvement, fibrosis and systemic scleroderma associated with systemic lupus erythematosus, polymyositis, dermatomyositis;
complications after surgery; diabetes; and cancer.

In addition, the diseases listed in Table 1 in Wynn TA., Cellular and molecular mechanisms of fibrosis, J Pathol 2008; 214: 199-210 can also be targets of treatment by the therapeutic agents of the present invention.

Furthermore, DNAs capable of expressing an siRNA of the present invention (double-stranded RNAs) or the RNA of any one of SEQ ID NOs: 1 to 4 are also included in the present invention. Thus, the present invention provides DNAs (vectors) capable of expressing an siRNA of the present invention (double-stranded RNAs). DNAs (vectors) capable of expressing an above-described double-stranded RNA of the present invention include, for example, DNAs having a structure in which a DNA encoding one of the strands of the double-stranded RNA and a DNA encoding the other strand of the double-stranded RNA are operably linked to a promoter to enable expression of both DNAs. Those skilled in the art can produce the above-described DNAs of the present invention using general genetic engineering techniques. More specifically, the expression vectors of the present invention can be produced by appropriately inserting DNAs encoding RNAs of the present invention (for example, the RNA of any one of SEQ ID NOs: 1 to 4) into various known expression vectors.

The sequence of CHST15 (GalNAc4S-6ST) of the present invention can be obtained, for example, based on Accession Number NM_015892. As an example, the nucleotide sequence of the CHST15 gene of the present invention is shown in SEQ ID NO: 5, and the amino acid sequence encoded by the gene is shown in SEQ ID NO: 6.

In addition to the proteins comprising the amino acid sequence shown above, the CHST15 protein of the present invention includes, for example, proteins having a high identity (typically 70% or higher, preferably 80% or higher, more preferably 90% or higher, and most preferably 95% or higher) with the sequence of SEQ ID NO: 6 and having a function of the above protein. The above protein is, for example, a protein comprising an amino acid sequence with an addition, deletion, substitution, or insertion of one or more amino acids in the amino acid sequence of SEQ ID NO: 6, in which the number of altered amino acids is typically 30 amino acids or less, preferably ten amino acids or less, more preferably five amino acids or less, and most preferably three amino acids or less.

The above-described gene of the present invention includes, for example, endogenous genes of other organisms which correspond to a DNA comprising the nucleotide sequence of SEQ ID NO: 5 (homologues to the above-described human gene or the like).

Each of the endogenous DNAs of other organisms which correspond to the DNA comprising the nucleotide sequence of SEQ ID NO: 5 are generally has a high identity (homology) with the DNA of SEQ ID NO: 5. High identity means preferably 70% or higher homology, more preferably 80% or higher homology, and still more preferably 90% or higher homology (for example, 95% or higher, or 96%, 97%, 98%, or 99% or higher). Homology can be determined using the mBLAST algorithm (Altschul, et al. Proc. Natl. Acad. Sci. USA, 1990, 87:2264-8; Karlin and Altschul, Proc. Natl. Acad. Sci. USA, 1993, 90:5873-7). When the DNAs have been isolated from the body, each of them may hybridize under stringent conditions to the DNA of SEQ ID NO: 5. Herein, "stringent conditions" include, for example, "2x SSC, 0.1% SDS, 50°C", "2x SSC, 0.1% SDS, 42°C", and "1x SSC, 0.1% SDS, 37°C"; more stringent conditions include "2x SSC, 0.1% SDS, 65°C", "0.5x SSC, 0.1% SDS, 42°C", and "0.2x SSC, 0.1% SDS, 65°C".

Meanwhile, the agents of the present invention can also be referred to as "pharmaceutical agents", "pharmaceutical compositions", "therapeutic medicines", or the like.

The "treatment" in the present invention also includes preventive effects that can suppress the onset of diseases in advance. Treatments are not limited to those having a complete therapeutic effect, and the effects may be partial.

The agents of the present invention can be combined with physiologically acceptable carriers, excipients, diluents and such, and orally or parenterally administered as pharmaceutical compositions. Oral agents may be in the form of granules, powders, tablets, capsules, solutions, emulsions, suspensions, or the like. The dosage forms of parenteral agents can be selected from injections, infusions, external preparations, inhalants (nebulizers), suppositories, and the like. Injections include preparations for subcutaneous, intramuscular, intraperitoneal, intracranial, and intranasal injections, and the like. The external preparations include nasal preparations, ointments, and such. Techniques for formulating the above-described dosage forms so as to contain the agents of the present invention as primary ingredients are known.

For example, tablets for oral administration can be produced by compressing and shaping the agents of the present invention in combination with excipients, disintegrants, binders, lubricants, and the like. Excipients commonly used include lactose, starch, mannitol, and the like. Commonly used disintegrants include calcium carbonate, carboxymethylcellulose calcium, and the like. Binders include gum arabic, carboxymethylcellulose, and polyvinylpyrrolidone. Known lubricants include talc, magnesium stearate, and such.

Known coatings can be applied to tablets containing the agents of the present invention to prepare enteric coated formulations or for masking. Ethylcellulose, polyoxyethylene glycol, or such can be used as a coating agent.

Meanwhile, injections can be prepared by dissolving the agents of the present invention, which are chief ingredients, together with an appropriate dispersing agent, or dissolving or dispersing the agents in a dispersion medium. Both water-based and oil-based injections can be prepared, depending on the selection of dispersion medium. When preparing water-based injections, the dispersing agent is distilled water, physiological saline, Ringer's solution or such. For oil-based injections, any of the various vegetable oils, propylene glycols, or such is used as a dispersing agent. If required, a preservative such as paraben may be added at this time. Known isotonizing agents such as sodium chloride and glucose can also be added to the injections. In addition, soothing agents such as benzalkonium chloride and procaine hydrochloride can be added.

Alternatively, the agents of the present invention can be formed into solid, liquid, or semi-solid compositions to prepare external preparations. Such solid or liquid compositions can be prepared as the same compositions as described above and then used as external preparations. The semi-solid compositions can be prepared using an appropriate solvent, to which a thickener is added if required. Water, ethyl alcohol, polyethylene glycol, and the like can be used as the solvent. Commonly used thickeners are bentonite, polyvinyl alcohol, acrylic acid, methacrylic acid, polyvinylpyrrolidone, and the like. Preservatives such as benzalkonium chloride can be added to these compositions. Alternatively, suppositories can be prepared by combining the compositions with carriers, like oil bases such as cacao butter, or aqueous gel bases such as cellulose derivatives.

When the agents of the present invention are used as gene therapy agents, the agents may be directly administered by injection, or vectors carrying the nucleic acid may be administered. Such vectors include adenovirus vectors, adeno-associated virus vectors, herpes virus vectors, vaccinia virus vectors, retroviral vectors, and lentivirus vectors. These vectors allow efficient administration.

In a preferred embodiment of the present invention, administration includes local administrations. Specifically, examples include method of injecting beneath the mucous membrane of the large intestine, rectum, and such using an endoscope (endoscopic local administration).

Alternatively, the agents of the present invention can be encapsulated into phospholipid vesicles such as liposomes, and then the vesicles can be administered. Vesicles carrying siRNAs or shRNAs are introduced into given cells by lipofection. The resulting cells are then systemically administered, for example, intravenously or intra-arterially.

The agents of the present invention are administered to mammals including humans at required (effective) doses, within a dose range considered to be safe. Ultimately, the doses of the agents of the present invention can be appropriately determined by medical practitioners or veterinarians after considering the dosage form and administration method, and the patient's age and weight, symptoms, and the like. Commercially available gene transfer kits (for example: AdenoExpress™, Clontech) may be used to introduce siRNAs or shRNAs into target tissues or organs.

The present invention also relates to methods for treating Crohn's disease or ulcerative colitis, which comprise the step of administering an RNA or DNA of the present invention or an agent of the present invention to an individual (for example, a patient) or a cellular tissue.

In the methods of the present invention, the individual is preferably a human but is not particularly limited thereto, and may also be a nonhuman animal.

In general, administration to an individual can be achieved by methods known to those skilled in the art, for example, local administration using an endoscope (for example, submucosal administration in the colon or rectum), intra-arterial injection, intravenous injection, or subcutaneous injection. The dose varies depending on the weight and age of the subject (patient or such), administration method, and so on; however, those skilled in the art can appropriately select a suitable dose.

In addition, the present invention relates to use of the RNAs or DNAs of the present invention or use of the agents of the present invention in the manufacture of an agent for treating Crohn's disease or ulcerative colitis.

All prior art documents cited herein are incorporated herein by reference.

### Examples

Hereinbelow, the present invention will be specifically described with reference to the EXAMPLES, but the technical scope of the present invention is not to be construed as being limited thereto.

"Anti-CHST15 siRNA" described in the EXAMPLES herein is an siRNA having a structure in which the RNAs of SEQ ID NOs: 3 and 4 are hybridized.

### [Objectives]

The anti-CHST15 siRNA was assessed for its therapeutic effect in chronic colitis model mice induced by dextran sulfate sodium (DSS). This model shows inflammatory fibrotic lesions in the mucosal and submucosal layers, which coincide with inflammatory fibrotic lesions accompanied by submucosal thickening observed in patients with Crohn's disease, and thus, the model can be used to evaluate the therapeutic effect on lesions.

### [Methods]

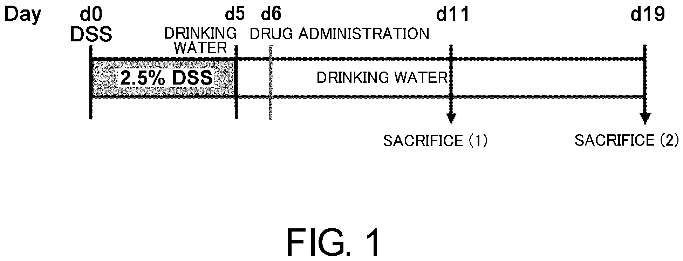

Drinking water was switched to regular water 6 days (from Day 5) after the start of 2.5% DSS consumption. On the seventh day (Day 6), the mice were administered in the submucosa of the large intestine with the anti-CHST15 siRNA at 25 nM, 250 nM, and 2500 nM per volume of the mouse large intestine (Fig. 1). Autopsies of the mice were carried out on the 12th day (Day 11) and 20th day (Day 19) to determine the level of the CHST15 gene expression and the hydroxyproline level in the large intestine. The details of the assay conditions are shown in Tables 1 to 6 below.

**Table 1**

| Assay system | |
|---|---|
| Species/Strain | C57BL/6J mice |
| Animal supplier | CLEA Japan Inc. |
| Age in weeks at the time of purchase | 8 weeks old |
| Age in weeks at the time of use | 10 weeks old |
| Sex | Female |
| Condition of keeping animals | SPF |

**Table 2**

| Method for incuding chronic colitis | |
|---|---|
| Solution administered on Days 0 to 6 | 2.5% DSS (average molecular weight: 36000 to 50000; Lot No.5237K, MP Biomedicals) was prepared using purified water (Millipore). Mice were allowed to drink DSS water from Day 0 and then regular drinking water from Day 5. Mice in the control group were allowed to drink purified water. All drinking water and solutions to be administered were freshly prepared once every week. |

**Table 3**

| Condition of inj ection | |
|---|---|
| Injected substance | Anti-CHST15 siRNA and negative control |
| Injection volume | 20 µL per volume of administered site in the large intestine |
| Reason for the injection volume | DSS was administered to an assumed volume of 0.225 cm³ (7.5 cm x 0.3 cm x 0.1 cm) adiministered site to induce inflammation throughout the large intestine. |

**Table 4**

| Experimental groups | | | | | |
|---|---|---|---|---|---|
| | Group | Dose/Large intestine | 2.5 % DSS | N (Experiment 1) | N (Experiment 2) |
| 1 | Non-treated | - | - | 6 | 3 |
| 2 | Control (Monitor) | - | + | 6 | 3 |
| 3 | Negative control (Control siRNA) | Negative control: 250 nM | + | 6 | 3 |
| 4 | Low-dose anti-CHST15 siRNA | anti-CHST15 siRNA: 25 nM | + | 6 | 3 |
| 5 | Moderate-dose anti-CHST15 siRNA | anti-CHST15 siRNA: 250 nM | + | 6 | 3 |
| 6 | High-dose anti-CHST15 siRNA | anti-CHST15 siRNA: 2500 nM | + | 6 | 3 |

**Table 5**

| Preparation of anti-CHST15 siRNA injection | |
|---|---|
| Substance | Anti-CHST15 siRNA (molecular weight: 17232.97 Da) |
| Injection route | Submucosa: Submucosally [3] injected at a rectal site 1.0 to 1.5 cm from the anus using an endoscope. |
| Injection dose | 25 nM, 250 nM, 2500 nM/0.225 mL |
| Concentration of injection | 0.005 mg/mL, 0.05 mg/mL, 0.5 mg/mL |
| Injection volume | 20 µL/head |
| Date of administration | Day 6 |
| Reason for the date of administration | On Day 6, DSS-induced colitis was endoscopically apparent. Histopathologically, edema and inflammatory fibrosis with thickening in the submucosal layer were prominent on Day 6. |

**Table 6**

| Preparation of injection for negative control | |
|---|---|
| Substance | 21-mer negative control siRNA (molecular weight: 13,315 Da, Lot No. 0003116, COSMO BIO) |
| Injection route | Submucosa: Submucosally [3] injected at a rectal site 1.0 to 1.5 cm from the anus using an endoscope. |
| Injection dose | 250 nM/0.225 mL |
| Concentration of injection | 0.05 mg/mL |
| Injection volume | 20 µL/head |
| Date of administration | Day 6 |
| Reason for the date of administration | Set to be identical to the administration of the anti-CHST15 siRNA. |
| Method for perparing injection | A stock solution (1.0 mg/mL negative control siRNA) was prepared using physiological saline (Otsuka Pharmceutical Factory Inc.), and then diluted 20 fold. |

The outline of the assay design is shown in Fig. 1. Specifically, first, C57BL/6J mice were administered with a 2.5% DSS solution (in each of the low-, moderate-, and high-dose anti-CHST15 siRNA groups, and control and negative control groups) or purified water (non-treated) between Day 0 and Day 5.

The body weight score, diarrheal stool score, and bloody stool score were observed every day between Day 0 and Day 6. The body weight score, diarrheal stool score, and bloody stool score were calculated according to Tables 7, 8, and 9, respectively. The total value is defined as the Disease Activity Score (DAI). The general conditions were observed every day between Day 0 and Day 19.

**Table 7**

| | Body weight score | | | | |
|---|---|---|---|---|---|
| | 0 | 1 | 2 | 3 | 4 |
| Reduction rate since Day 0 | >1% | 1-5% | 5-10% | 10-20% | >20% |

**Table 8**

| | Diarrheal stool score | | | | |
|---|---|---|---|---|---|
| | 0 | 1 | 2 | 3 | 4 |
| Stool type | Normal | - | Loose stool | - | Diarrhea |

**Table 9**

| | Bloody stool score | | | | |
|---|---|---|---|---|---|
| | 0 | 1 | 2 | 3 | 4 |
| Score based on test strips | - | + | ++ | +++ | Continuous bleeding from the anus |

Photographs of all the animals were taken using an endoscope on the day of administration (Day 6) and days of sacrifice (Day 11 and Day 19). Three photographs were taken at distances of 0.5 cm, 1.0 cm, and 1.5 cm from the anus in each animal to assess the effect of the anti-CHST15 siRNA on colitis. All photographs of Day 6 and Day 19 were scored by the segmental SES-CD scoring method. The SES-CD score was developed as a highly sensitive endoscopic index system, and has been used in clinical diagnosis. The criteria in the segmental SES-CD scoring are shown in Table 10.

**Table 10**

| | Segmental SES-CD score | | | |
|---|---|---|---|---|
| | 0 | 1 | 2 | 3 |
| Ulcer size | None | Aphthous ulcer | Ulcer | Large ulcer |
| Degree of ulcerated surface | None | Less than 10% | 10-30% | More than 30% |
| Degree of severe ulcer | None | Less than 50% | 50-75% | More than 75% |
| Stricture type | None | Single site | Multiple, passable | Unpassable |

In the experiments described above, an assessment by the segmental SES-CD scoring gave a total score of 12 for sites that are 0.5 to 1.5 cm from the anus in the large intestine.

### [Conclusive observation on Day 11 and Day 19]

### 1. Blood sampling method

Blood was collected under the conditions described in Table 11 below.

**Table 11**

| Anesthesia | Inhalation anesthesia with diethyl ether |
|---|---|
| Route of anesthesia | Inhalation |
| Route of blood sampling | Blood sampling from the heart |
| Blood sampling apparatus | 1-mL syringe with 23-G needle (Terumo) and eppendorf tube Separation by centrifugation (Centrifuge 5417R, Eppendorf) |

### 2. Methods for analyzing gene expression

### (1) RNA extraction

A rectal sample for use in gene expression analysis was soaked in RNAlater (Lot No. 108K0483, SIGMA Aldrich) and stored at 4°C. The sample was homogenized using RNAiso Plus (Takara). After phenol/chloroform treatment, the collected lysate was loaded onto a cartridge of the RNA fast pure kit (Lot No. 1001, Takara), and centrifuged at 8000 x g at room temperature for one minute to allow adsorption of nucleic acids onto the cartridge. After 750 µL of 10% EtOH WB solution was loaded onto the cartridge, the cartridge was washed by centrifugation at 8000 x g at room temperature for one minute. To elute the total RNA from the cartridge, 30 µL of EB solution was loaded; and after four minutes of incubation at room temperature, the cartridge was centrifuged at 8000 x g at room temperature for one minute. The concentration of the prepared total RNA was calculated based on the measured absorbance of the total RNA (1 OD = 40 µg/mL total RNA).

### (2) Reverse transcription reaction and real-time RT-PCR reaction

The total RNA solution was diluted to 1000 ng/µL to prepare samples for quantitation, and reverse transcription was carried out using random 6-mer primers under the reaction conditions described in the Table below. Using the prepared cDNAs as template, relative quantitative real-time RT-PCR was carried out using real-time PCR primers against the target gene (mouse CHST15). For the standard curve, a four-fold dilution series starting from 10 ng/µL was prepared using cDNAs from the negative control group. Moreover, the same samples were assayed in the same manner using primers against mouse β-actin. The expression of the mouse CHST15 gene was normalized with the expression level of β-actin, and shown as a relative expression level by taking that of the non-treated group as 1. The real-time RT-PCR reaction was carried out using the real-time RT-PCR apparatus Thermal Cycler Dice Real Time System, following the protocol of SYBR Premix ExTaq II (Perfect Real Time) under the reaction conditions described in Tables 12 and 13.

**Table 12**

| PCR REACTION CONDITIONS - REVERSE TRANSCRIPTION REACTION | | | |
|---|---|---|---|
| Reagent | Amount used (µL)/Reaction | Final concentration | Reaction condition |
| 5 x M-MLVRT buffer | 4 | - | 37°C: 60 min |
| M-MLV Reverse | 1 | 10 U/µL | 99°C: 5 min |
| Transcriptase | | | 4°C |
| Random Primers | 0.5 | 6.28 µM | |
| 10 mM dNTP Mix | 1 | 0.5 mM | |
| 40 U/µL RNase inhibitor | 1 | 2 U/µL | |
| 25 mM MgCl₂ | 3.5 | 10 mM | |
| 0.1 MDTT | 2 | 10 mM | |
| Total RNA + RNase Free water | 7 | - | |
| Total | 20 | - | |

**Table 13**

| PCR REACTION CONDITIONS - REALTIME RT-PCR REACTION | | | | |
|---|---|---|---|---|
| Reagent | Amount used (µL)/Reaction | Final concentration | Reaction condition | |
| SYBR Premix Ex Taq II (2x) | 12.5 | 1x | 95°C: 30 sec | Cycle: 1 |
| PCR Forward Primer | 1 | 0.2 µM | 95°C: 5 sec | Cycle: 40 |
| (10 µM) | | | 60°C: 45 sec | Cycle: 40 |
| PCR Reverse Primer (10 µM) | 1 | 0.2 µM | Dissociation Curve | |
| RT reaction solution (cDNA solution) + dH₂O (sterilized distilled water) | 10.5 | | | |
| Total | 25 | - | | |

### 3. Method for measuring hydroxyproline

After weighing the rectal samples for use in hydroxyproline assay, 2N NaOH was added and the samples were heat-treated in Block incubator (ASTEC) at 65°C for 10 minutes. The amount of protein was measured using 10 µL of the intestine lysates by using the BCA assay kit (Lot No. LD144450, Thermoscientific), and the remaining lysates were autoclaved at 121°C for 20 minutes to carry out thermal alkaline degradation. 6N HCl was added to the thermal alkaline degradation samples; and the samples were autoclaved for 20 minutes at 121°C, and then hydrolyzed and neutralized by adding an activated carbon-4N NaOH solution and acetate-citrate buffer. The resulting samples were centrifuged to collect the supernatants. A chloramine T reagent was added to the supernatants, mixed, and incubated at room temperature for 25 minutes. Then, Ehrlich's reagent was added, mixed, heated at 65°C for 20 minutes, and then cooled for five minutes on ice. After centrifugation at 4°C, 150 µL of the obtained supernatants were aliquoted into a 96-well plate, and the absorbance at 560 nm was measured using Power wave XS (Biotek). The level of hydroxyproline in the samples was calculated by the 4-parametric method from the standard curve prepared using standard hydroxyproline solutions. The obtained hydroxyproline levels were normalized with the protein concentrations.

### [Statistical analysis]

The assay result is shown as mean ± SD. All tests were performed using the Bonferroni multiple comparison test (PRISM software). p<0.05 was considered statistically significant.

### [Results]

### 1. General symptoms

In all groups except the non-treated group, piloerection was observed after Day 1 or 2. Other than the relevant pathological conditions, no other abnormalities were seen in terms of general symptoms in any animal during the test period.

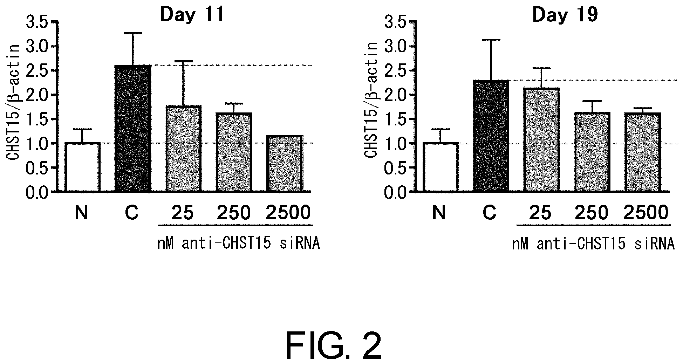

### 2. Gene expression analysis (Fig. 2)

When compared to the non-treated group (N), the control group and the negative control group (C) showed increases in the expression level of the CHST15 gene both on Day 11 and Day 19. The expression level of the CHST15 gene in the anti-CHST15 siRNA-administered groups was decreased as compared to that in the negative control group. A tendency of concentration dependence was also observed on Day 19.

In Table 14, the average knockdown rate (%) of the anti-CHST15 siRNA is shown with the expression level of the CHST15 gene in the non-treated group (N) being the baseline (0%) and the mean value in the negative control group (C) being 100%.

**Table 14**

| anti-CHST15 siRNA concentration | 25 nM | 250 nM | 2500 nM |
|---|---|---|---|
| Day 11 | 43% | 53% | 89% |
| Day 19 | 14% | 55% | 55% |

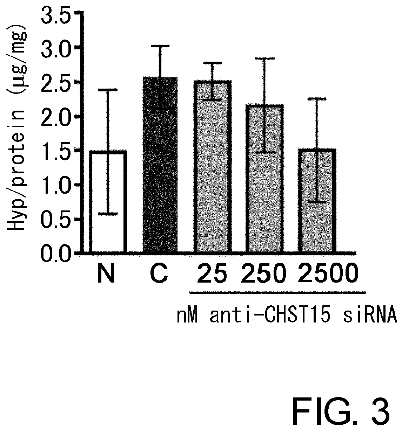

### 3. Measurement of hydroxyproline (Fig. 3)

When compared to the non-treated group (N), the control group and the negative control group (C) showed a tendency of increasing hydroxyproline level in the large intestine both on Day 11 and Day 19. In the anti-CHST15 siRNA-administered groups (250 nM and 2500 nM), the hydroxyproline level in the large intestine was decreased on Day 19. A tendency of concentration dependence was also observed.

With the mean value of the hydroxyproline level in the large intestine in the negative control group (C) being taken as 100%, the average rate of decrease (%) by the anti-CHST15 siRNA is as shown in Table 15.

**Table 15**

| anti-CHST15 siRNA concentration | 25 nM | 250 nM | 2500 nM |
|---|---|---|---|
| Day 19 | 7% | 17% | 42% |

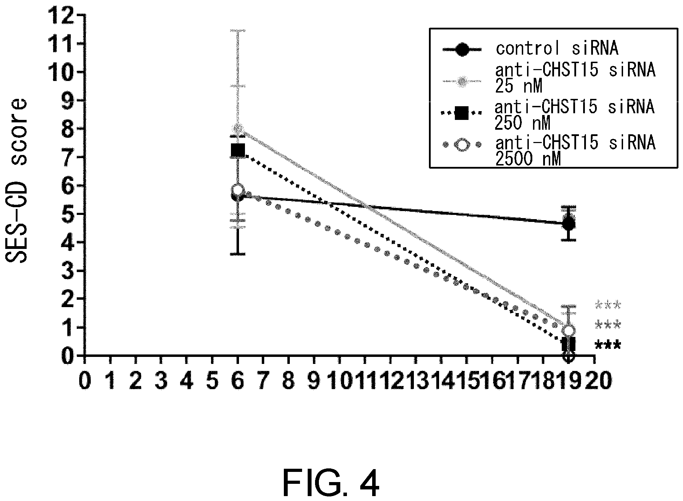

### 4. Segmental SES-CD score (Fig. 4)

In the negative control group, there is a tendency of decreasing segmental SES-CD score on Day 19 as compared to Day 6. On Day 19, the segmental SES-CD score was found to be significantly lower in the anti-CHST15 siRNA-administered groups at all concentrations as compared with the negative control group. Concentration dependency was not clear.

### [Conclusions]

In 2.5% DSS-induced chronic colitis model mice, when the anti-CHST15 siRNA was administered at a dose of 25 nM, 250 nM, or 2500 nM in the submucosa of the large intestine, the expression level of the CHST15 gene was reduced although there was no clear concentration dependency. When the anti-CHST15 siRNA was used at 250 nM or 2500 nM, the hydroxyproline level in the large intestine was decreased on Day 19, and thus, an anti-fibrosis effect was demonstrated by suppressing the expression of the CHST15 gene (target gene).

### Industrial Applicability

The anti-CHST15 siRNAs of the present invention produced the effects of suppressing the CHST15 gene expression and reducing the hydroxyproline level in the colon. Furthermore, they exhibited the effect of markedly suppressing the segmental SES-CD score, which is the most clinically important point of observation. This result suggests that the anti-CHST15 siRNAs of the present invention are clearly effective in improving endoscopic lesions caused by inflammation and fibrosis in the chronic colitis model mice.

### Aspects of the invention include:

1. An siRNA that suppresses expression of the CHST15 gene, which comprises a structure in which an RNA comprising the nucleotide sequence of SEQ ID NO: 1 is hybridized to an RNA comprising a complementary sequence thereof.
2. The siRNA of item 1, which has a structure with an overhang of one or more nucleic acids at one end.
3. A DNA vector capable of expressing the siRNA of item 1 or 2.
4. An agent for promoting mucosal healing, which comprises as an active ingredient the siRNA of item 1 or 2 or the DNA of item 3.
5. An agent for treating Crohn's disease or ulcerative colitis, which comprises as an active ingredient the siRNA of item 1 or 2 or the DNA of item 3.
6. A pharmaceutical composition for wound healing, mucosal healing, or ulcer healing, which comprises as an active ingredient the siRNA of item 1 or 2 or the DNA of item 3.

## Claims

1. An agent for use in treating a patient with Crohn's disease or ulcerative colitis, which comprises as an active ingredient
(i) an siRNA molecule in which the nucleotides at the 3'-end overhang are AG and AU having the following structure: 
5' -GGAGCAGAGCAAGAUGAAUACAAUCAG-3'    (SEQ ID NO: 3)
IIIIIIIIIIIIIIIIIIIIIIIIII
3' -UACCUCGUCUCGUUCUACUUAUGUUAG-5'    (SEQ ID NO: 4)
(ii) or a DNA vector capable of expressing the siRNA molecule defined in item (i).

2. The agent for use according to claim 1 for mucosal healing.

3. The agent for use according to claim 1, wherein the patient has endoscopic lesions.

4. The agent for use according to claim 1, wherein the patient has inflammatory fibrotic lesions in the mucosal and submucosal layers accompanied by submucosal thickening observed in the patient with Crohn's disease.

5. The agent for use according to any one of claims 1-3, wherein the agent is to be administered by local administration using an endoscope.

6. The agent for use according to claim 4, wherein the agent is to be administered by submucosal administration in the colon or rectum.
